# EUROPÄISCHE PATENTANMELDUNG

(11) **EP 1 402 839 A1**
(43) Veröffentlichungstag der Anmeldung: **31.03.2004**
(21) Anmeldenummer: 03017215.9
(22) Anmeldetag: 29.07.2003
(51) Int. Cl.: A61B 19/00

(54) **Lageindikator**

(30) Priorität: 27.09.2002 DE 10245287
(71) Anmelder: Mathys Medizinaltechnik AG, 2544 Bettlach (CH)
(72) Erfinder: Christen, Peter, 2545 Selzach (CH); Fankhauser, Christoph, 4500 Solothurn (CH); Delfosse, Daniel, 3018 Bern (CH)
(74) Vertreter: Körfer, Thomas, Dipl.-Phys.

(57) **Zusammenfassung**

Ein Lageindikator (1) zur Visualisierung der Lage sowie Lageveränderungen eines Skelettteils während eines operativen Eingriffs weist einen Körper (2) auf, welcher zumindest eine Anschlagkante (3) und ein in dem Körper (2) angeordnetes Kugelgelenk (9) umfaßt, in welchem ein Fixierungselement (5) gelagert ist.

## Beschreibung

Die Erfindung betrifft eine Vorrichtung zur Indikation der Lage eines menschlichen oder tierischen Knochens, der während einer Operation meist von Weichteilen teilweise verdeckt ist.

Zur Lageindikation bei operativen Eingriffen, wie z. B. Hüftgelenksimplantationen, werden heute zur Verbesserung der räumlichen Vorstellung über die Lage der Knochen, beispielsweise des Beckens, und der Implantate, beispielsweise einer Hüftgelenkspfanne, typischerweise Navigationssysteme mit aufwendiger Infrastruktur eingesetzt. Hierbei kann es sich z. B. um ein Kamerasystem handeln, welches Referenzbasen an den Operationsinstrumenten sowie eine umfangreiche Hard- und Softwareausstattung erfordert.

Beispielsweise ist aus der AT 404 092 B ein medizinisches Navigationssystem zum dreidimensionalen Erfassen der Lage von Meßpunkten an einem Patienten und zur Bildschirmdarstellung des jeweiligen Meßpunktes in vorher angefertigten Schnittbildern und/oder dreidimensionalen Bildern desselben Patienten bekannt, wobei das Navigationssystem einen mit mehreren Gelenken versehenen Arm aufweist, dessen freies Ende in Position und Winkellage erfaßt wird.

Nachteilig an den bekannten Systemen ist dabei insbesondere, daß die Kosten für die Anschaffung und Wartung eines derartigen Systems und des benötigten Zubehörs sowie der Aufwand beim Einsatz während der Operation sehr hoch sind. Insbesondere letzteres kann die Operationsdauer verlängern.

Der Erfindung liegt demnach die Aufgabe zugrunde, eine Vorrichtung zu schaffen, welche sowohl kostengünstig ist als auch eine einfache und schnelle Lageindikation des zu operierenden Knochens sowie der Implantate ermöglicht.

Die Aufgabe wird durch die Merkmale des Anspruchs 1 gelöst.

Verbesserungen und Weiterbildungen der Erfindung sind in den Unteransprüchen angegeben.

Ein besonders vorteilhaftes Ausführungsbeispiel des Lageindikators zeichnet sich durch einen würfelförmigen Körper mit drei zueinander senkrechten Anschlagkanten aus, welche ein kartesisches Koordinatensystem aufspannen und somit die Ausrichtung im Raum ermöglichen.

Vorteilhafterweise umfaßt der Lageindikator einen Fixierungsdraht, der in den Knochen eingebracht wird. Im Ausführungsbeispiel ist der Fixierungsdraht in den Knochen eingeschraubt. Lageänderungen des Knochens werden somit auf den Lageindikator in einfacher Weise übertragen.

Weiterhin ist von Vorteil, daß durch ein Kugelgelenk ein frei wählbarer Winkelbereich definiert ist, innerhalb dessen der würfelförmige Körper relativ zum Fixierungsdraht in seiner Position fixierbar ist.

Vorteilhafterweise können Verlängerungen, welche parallel zu den Anschlagkanten verlaufen, an dem Gehäuse angebracht werden, wodurch die Lage des Knochens deutlicher dargestellt werden kann. Im Ausführungsbeispiel sind die Verlängerungen in Form von Verlängerungsdrähten ausgebildet.

Die Erfindung wird im folgenden anhand eines bevorzugten Ausführungsbeispiels anhand der Zeichnung näher erläutert. In der Zeichnung zeigen:
- Fig. 1: eine perspektivische Gesamtansicht eines Ausführungsbeispiels der erfindungsgemäßen Vorrichtung, und
- Fig. 2: eine teilweise geschnittene Ansicht des in Fig. 1 dargestellten Ausführungsbeispiels.

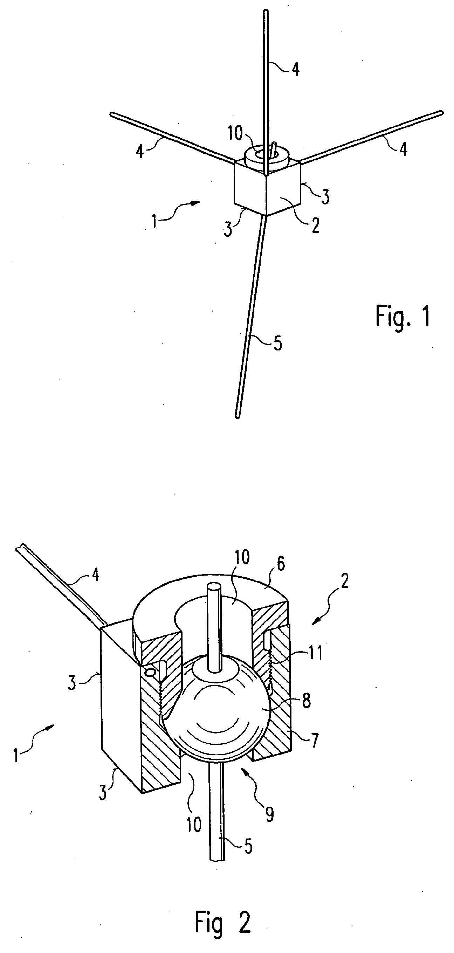

Fig. 1 zeigt in einer schematischen Darstellung ein Ausführungsbeispiel eines erfindungsgemäß ausgestalteten Lageindikators 1. Der Lageindikator 1 weist dabei einen Körper 2 auf, welches im vorliegenden Ausführungsbeispiel würfelförmig ausgebildet ist. Gemäß seiner würfelförmigen Form weist der Körper 2 drei Anschlagkanten 3 auf, welche rechtwinklig zueinander stehen. Der Lageindikator 1 ist mittels der Anschlagkanten 3 in beliebiger, festgelegter Position relativ zu einem Bezugssystem wie z. B. einem Operationstisch anlegbar. Er kann mittels einer geeigneten Vorrichtung fixiert werden.

Parallel zu den Anschlagkanten 3 sind Verlängerungen 4 angeordnet, welche in den Körper 2 einsteckbar sind und sich in beliebiger Ausdehnung erstrecken können. Die Verlängerungen 4 sind im Ausführungsbeispiel als Verlängerungsdrähte 4 ausgebildet.

In den würfelförmigen Körper 2 ist ein Fixierungselement in Form eines Fixierungsdrahts 5 geführt. Der Fixierungsdraht 5 wird bei Operationsbeginn in dasjenige Skelettteil, dessen Lage visualisiert werden soll, beispielsweise eingeschraubt und verändert seine Lage entsprechend der Lageveränderung des Skelettteils während des Operationsverlaufs. Der Fixierungsdraht 5 ist dabei vorzugsweise aus chirurgischen Werkstoffen wie z. B. Stahl oder Kunststoff hergestellt und kann insbesondere als sogenannter Kirschnerdraht ausgebildet sein. Lageänderungen des Skelettteils während der Operation sind durch die Veränderung des Koordinatensystems, aufgespannt durch die Verlängerungen 4, erkennbar. Die Ausrichtung von in das Skeletteil einzubringenden Implantaten kann in einfacher Weise kontrolliert und korrigiert werden.

Fig. 2 zeigt in einer schematischen, teilweise geschnittenen Ansicht die Führung des Fixierungsdrahtes 5 in dem würfelförmigen Körper 2.

Der würfelförmige Körper 2 ist zweiteilig aufgebaut und umfaßt zwei Lagerkörper 6 und 7, in welchen eine Kugel 8 gelagert ist. Der Fixierungsdraht 5 durchgreift dabei sowohl die Kugel 8 als auch die Lagerkörper 6, 7. Letztere weisen dabei Öffnungen 10 auf, welche eine festgelegte Weite besitzen. Diese ist dabei so gewählt, daß sich der Fixierungsdraht 5 in einem festgelegten Winkelbereich bewegen kann. Dieser beträgt beispielsweise zwischen einer achsparallelen Stellung des Fixierungsdrahtes 5 relativ zum Körper 2 und dem Anschlag des Fixierungsdrahtes 5 am Rand der Öffnung 10 zwischen 0° und 45° mit einem bevorzugten Winkelbereich von 0° bis 25°.

Eine Klemmvorrichtung erlaubt die Fixierung des Fixierungsdrahtes 5 in einer frei wählbaren Position. Im Ausführungsbeispiel wird die Klemmvorrichtung durch die an einem Gewinde 11 miteinander verschraubten Lagerkörper 6 und 7 gebildet. Werden die Lagerkörper 6 und 7 aufeinander zu geschraubt, verjüngt sich der sphärische Lagerraum für die Kugel 8 und die Kugel 8 wird zwischen den Lagerkörpern 6 und 7 fixiert.

Eine ebenfalls nicht dargestellte Skala auf dem Lagerkörper 6 im Bereich der Öffnung 10 erlaubt die Quantifizierung der Lageveränderung des Patienten.

Die Erfindung ist nicht auf das dargestellte Ausführungsbeispiel beschränkt. Beispielsweise kann der Körper 2 auch mit nur einer Anschlagkante 3 versehen sein, wenn eine Positionsbestimmung nur in einer Richtung ausreichend ist. Die Weite der Öffnungen 10 des Körpers 2 kann an die Erfordernisse des Skelettteils angepaßt werden.

## Patentansprüche

1. Lageindikator (1) zur Visualisierung der Lage sowie von Lageveränderungen eines Skelettteils während eines operativen Eingriffs, wobei der Lageindikator (1) einen Körper (2) aufweist, welcher zumindest eine Anschlagkante (3) und ein in dem Körper (2) angeordnetes Kugelgelenk (9) umfaßt, in welchem ein Fixierungselement (5) gelagert ist.

2. Lageindikator nach Anspruch 1,
**dadurch gekennzeichnet,**
**daß** der Körper (2) würfelförmig ausgebildet ist und drei Anschlagkanten (3) aufweist.

3. Lageindikator nach Anspruch 2,
**dadurch gekennzeichnet,**
**daß** die Anschlagkanten (3) ein dreidimensionales kartesisches Koordinatensystem aufspannen.

4. Lageindikator nach einem der Ansprüche 1 bis 3,
**dadurch gekennzeichnet,**
**daß** der Körper (2) zwei Lagerkörper (6, 7) bildet.

5. Lageindikator nach Anspruch 4,
**dadurch gekennzeichnet,**
**daß** eine Kugel (8) des Kugelgelenks (9) in den Lagerkörpern (6, 7) gelagert ist.

6. Lageindikator nach Anspruch 5,
**dadurch gekennzeichnet,**
**daß** der Fixierungsdraht (5) die Lagerkörper (6, 7) durch Öffnungen (10) durchgreift.

7. Lageindikator nach Anspruch 5 oder 6,
**dadurch gekennzeichnet,**
**daß** die Kugel (8) von dem Fixierungselement (5) durchgriffen ist.

8. Lageindikator nach Anspruch 7,
**dadurch gekennzeichnet,**
**daß** die Kugel (8) in den Öffnungen (10) der Lagerkörper (6, 7) in einem Winkelbereich von 0°bis 45° schwenkbar ist.

9. Lageindikator nach Anspruch 8,
**dadurch gekennzeichnet,**
**daß** der bevorzugte Winkelbereich von 0° bis 25° reicht.

10. Lageindikator nach Anspruch 8 oder 9,
**dadurch gekennzeichnet,**
**daß** das Fixierungselement (5) in seiner Winkelposition mittels einer Schraubverbindung arretierbar ist.

11. Lageindikator nach Anspruch 10,
**dadurch gekennzeichnet,**
**daß** die Schraubverbindung durch die miteinander verschraubten Lagerkörper (6, 7) gebildet ist.

12. Lageindikator nach einem der Ansprüche 1 bis 11,
**dadurch gekennzeichnet,**
**daß** an den Anschlagkanten (3) des würfelförmigen Gehäuses (2) Verlängerungen (4) fixiert sind.

13. Lageindikator nach einem der Ansprüche 1 bis 12,
**dadurch gekennzeichnet,**
**daß** das Fixierungselement (5) aus chirurgischen Werkstoffen wie Stahl oder Kunststoff besteht und insbesondere ein Kirschnerdraht ist.
